# EUROPEAN PATENT APPLICATION

(11) **EP 2 090 562 A1**
(43) Date of publication of application: **19.08.2009**
(21) Application number: 08465002.7
(22) Date of filing: 06.02.2008
(51) Int. Cl.: C07C 37/20, C07C 39/16

(54) **A method to obtain polycarbonate-grade bisphenol A**

(71) Applicant: Kiedik, Maciej, 47-220 Kedzierzyn-Kozle (PL); Instytut Inzynierii Materialow Polyimerowych i Barwnikow, 87-100 Torun (PL)
(72) Inventor: Kiedik, Maciej, 47-220, Kedzierzyn-Kozle (PL); Sokolowski, Adam, 53-030, Wroclaw (PL); Kolt, Jozef, 41-800, Zabrze (PL); Rzodeczko, Anna, 47-220, Kedzierzyn-Kozle (PL); Kubica, Stefan, 41-800, Zabrze (PL); Mroz, Jerzy, 47-220, Kedzierzyn-Kozle (PL); Chrusciel, Arkadiusz, 47-400, Raciborz (PL); Kaledkowska, Malgorzata, 47-220, Kedzierzyn-Kozle (PL)
(74) Representative: Piela, Marek

(57) **Abstract**

Polycarbonate-grade bisphenol A is obtained by condensing phenol with acetone in the presence of a cation exchanger either in two or in four steps that are effected either in one or in two reactors which are divided into reaction zones by means of sets of filtration-injection nozzles that are located at not less than three heights. Two reaction steps are effected in a single reactor, Step I being effected in the upper zone of the first reactor while the reaction mixture is made to flow downwards, whereas Step II is effected in the lower zone of the first reactor while the reaction mixture is made to flow upwards in the reactor.

The four reaction steps are effected in two reactors with filtration slot nozzles, Steps I and II being effected in the first reactor as described above whereas Steps III and IV are effected in the second reactor while the reaction mixture is made to flow either upwards or downwards in the reactor.

Bisphenol A from the post-reaction mixture is purified and separated by way of distillation and by falling-film melt fractional crystallization or by suspension crystallization.

## Description

### Technical Field

This invention relates to a method to obtain bisphenol A with high-purity, suitable for manufacturing colorless polycarbonates.

### Background Art

The US Patent No. 5315042 describes a continuous process to manufacture bisphenol A, by contacting phenol with acetone in the presence of an acid catalyst, at increased flowrates through the catalyst, the said flowrates being sufficient to reduce the conversion of acetone whereby the content of acetone is kept at a high level, accounting for a high reaction rate and a suitable increase in the yield of bisphenol A. Owing to this, the residence time of bisphenol A in the reaction zone is reduced and the content of byproducts affecting the coloration of bisphenol A is reduced as well. The method has the disadvantage of being too energy consuming, which results from the low conversion of acetone and adequately low (10-13 %) increase in the concentration of bisphenol A in the reaction, whereby the amount of unreacted raw materials for processing and for regeneration per 1 ton of product is very high.

The International Patent No. WO/19302 of China Petro-Chemical-Corp. describes a method to obtain bisphenol A, according to which the reaction is carried out using a phenol-to-acetone ratio by mole of ab. 4 to ab. 12, in the presence of a modified ion-exchange resin catalyst in an essentially vertical, multistage suspension stripping apparatus. The entire phenol required for the reaction of condensation with acetone is fed to the tower-reactor at a point over the top plate, part of the entire acetone required is fed to the plate below the top one and to some or all of the plates located at lower levels, whereas an inert gas stream is made to flow upwards through a catalytic chamber, thus forming a liquid- solid suspension, to strip off water from the reaction mixture. Technically, the method is highly complex and impracticable.

The Polish Patent Application P - 347705 describes a process carried out with the use of a double-zone catalytic bed, combined with post-reaction mixture take-off methods that are appropriate for such zones, and with keeping sufficiently high concentrations of water and appropriate temperatures in the catalytic bed, as well as with appropriate purification of the resulting product. Moreover, the process is characterized by low increase in the concentration of bisphenol A in the reaction, due to which the consumption of energy in the process is too high.

The European Patent No. EP 05777840.9 describes a method in which preliminary conversion to obtain an increased concentration of bisphenol A by 2 - 15 %, preferably 3 - 5 %, is effected in the condensation reaction Step I while in Step II the reaction is carried out so that the difference in water concentrations between the reactor inlet and outlet in Step II is kept in the range 0.1 - 0.5 %, preferably 0.1 - 0.2 %, while the difference in acetone concentrations between the reactor inlet and outlet in Step II is kept at a maximum of 2 %, preferably 1 %, the difference between the concentrations of bisphenol A between the reactor inlet and outlet is kept in the range 1 - 5 %, preferably 1 - 2 %, and the temperature difference between the reactor inlet and outlet in Step II is kept at a maximum of 15 ° C, preferably 5 °C.

Part of reaction water is removed from the reaction mixture in condensation Step II in a continuous manner. The post-reaction mixture resulting from Step II, which contains not less than 15 %, preferably not less than 23 %, of bisphenol A is sent to a distillation system to distill off a fraction that contains acetone, water and phenol, whereby crude bisphenol A that contains not more than 5 %, preferably not more than 1 %, of phenol is obtained as a residue. The crude bisphenol A is separated by distillation or is sent to melt fractional crystallization to obtain visually pure bisphenol A and a residue that contains bisphenol A, its isomers and other byproducts. The post-reaction mixture from the condensation reaction Step II is optionally sent to a suspension crystallization unit to obtain a crystalline bisphenol A-phenol adduct in the form of a suspension which is then subjected to filtration or centrifugation to separate the adduct which is then sent to a distillation system in order to separate bisphenol A.

After distilling off water, the post-crystallization liquor is sent back to the reaction or to a distillation system for separation. The residue after the distillation separation or after the fractional crystallization is diluted with the stream of phenol and is subjected to static crystallization, to obtain a bisphenol A-phenol adduct, which is sent to the distillation system together with the post-reaction mixture stream from the condensation reaction Step II. The residue after the static crystallization is carried outside the process after phenol has been distilled off. The Step II reactors and, optionally, the Step I reactors are divided each into at least two reaction sections by at least three sets of slot nozzles, located at various heights of the reactors. From one to six Step II reactors fall to every Step I reactor. The method of the invention enables visually pure bisphenol A to be obtained with selectivities up to 98 %.

### Disclosure of Invention

Technical Problem

It was the purpose of the invention to develop a method to obtain visually pure bisphenol A with high purity and with selectivities above 95 % so as to obtain an increase in the concentration of bisphenol A of more than 20 % in the reaction unit, whereby the energy consumption of the process to obtain bisphenol A is reduced.

Technical Solution

In the method of the invention, bisphenol A is obtained from phenol and acetone in the presence of an acid ion-exchanger catalyst in two reaction steps in a single reactor that is divided into reaction zones by means of sets of filtration-injection nozzles, located at not less than three heights. In Step I, the reaction is made to occur in the upper zone of the reactor while the reaction mixture is made to flow downwards to obtain an increase in the concentration of bisphenol A by 2 - 15 %, while Step II is made to occur in the lower zone of the reactor while the reaction mixture is made to flow upwards in the reactor so that the difference in the concentrations between the inlet to the reaction zone and the outlet: is 0.1 - 0.6 % for water, is a maximum of 2 % for acetone, is 1 - 6 % for bisphenol A, while the difference in temperatures between the inlet to the reaction zone and the outlet is a maximum of 8 °C, preferably a maximum of 3 °C.

In a preferable embodiment of the present invention, the reaction Step I is fed with a dewatered post-crystallization liquor resulting from centrifugation or filtration of the bisphenol A-phenol adduct, a post-reaction mixture from another reactor, or part of the feed mixture to the reaction Step II.

In another preferable embodiment of the present invention, bisphenol A is obtained from phenol and acetone in the presence of an acid ion-exchanger catalyst in four reaction steps, carried out in two reactors that are divided into reaction zones by means of sets of filtration-injection nozzles that are located at not less than three heights. Steps I and II proceed in the first reactor as described hereinabove, whereas Step III proceeds in the lower zone of the second reactor, while the reaction mixture is made to flow upwards in the reactor, so that the difference in the concentrations between the inlet to the reaction zone and the outlet: is 0.1 - 0.4 % for water, is a maximum of 2 % for acetone, is 1 - 4 % for bisphenol A, while the difference in temperatures between the inlet to the reaction zone and the outlet is a maximum of 5 °C, preferably a maximum of 2 °C. After Step III, part of water is removed from the reaction mixture by distillation to obtain a water concentration of not more than 1.3 %, preferably not more than 0.8 %, while adding acetone to obtain a concentration in the range from 2 to 4 %, and contacting the reaction mixture at a temperature in the range 70 - 90 °C with the cation exchanger, which is located in the upper zone of the second reactor, while the reaction mixture is made to flow downwards.

In another preferable embodiment of the present invention, bisphenol A is obtained from phenol and acetone in the presence of an acid ion-exchanger catalyst in four reaction steps, carried out in two reactors which are divided into reaction zones by means of sets of filtration-injection nozzles that are located at not less than three heights. Steps I and II proceed in the first reactor as described hereinabove, Step III proceeds in the upper zone of the second reactor, while the reaction mixture is made to flow downwards to obtain an increase in the concentration of bisphenol A by 1 - 5 %, whereas Step IV proceeds in the lower zone of the second reactor while the reaction mixture is made to flow upwards in the reactor, so that the difference in the concentrations between the inlet to the reaction zone and the outlet: is 0.1 - 0.3 % for water, is a maximum of 2 % for acetone, is 1 - 4 % for bisphenol A while the difference in temperatures between the inlet to the reaction zone and the outlet is a maximum of 6 °C, preferably a maximum of 2 °C.

In yet another preferable embodiment of the invention, bisphenol A is obtained from phenol and acetone in the presence of an acid ion-exchanger catalyst in four reaction steps, carried out in two reactors which are divided into reaction zones by sets of filtration-injection nozzles that are located at not less than three heights. Steps I and II proceed in the first reactor as described hereinabove, Step III proceeds in the lower zone of the second reactor while the reaction mixture is made to flow upwards in the reactor so that the difference in the concentrations between the inlet to the reaction zone and the outlet: is 0.1 - 0.4 % for water, is a maximum of 2 % for acetone, is 1 - 4 % for bisphenol A, while the difference in temperatures between the inlet to the reaction zone and the outlet is a maximum of 5 °C, preferably a maximum of 2 °C, whereas Step IV proceeds in the upper zone of the second reactor while the reaction mixture is made to flow upwards at a temperature in the range 74 - 90 °C.

In yet another preferable embodiment of the present invention, the resulting post-reaction mixture, containing from 23 to 30 % bisphenol A, is sent to a distillation system, to distill off acetone, water, and phenol, obtaining a residue in the form of crude bisphenol A, that contains not more than 5 %, preferably not more than 1 % phenol. The resulting product is fed to a falling-film melt-fractional crystallization unit, to obtain bisphenol A with high purity and a residue that contains bisphenol A, its isomers, and other byproducts whereas the distillate that contains acetone, water and phenol is separated by distillation to obtain acetone and phenol which are sent back to the process, and water which is removed from the process.

In another preferable embodiment of the present invention, after contacting the resulting post-reaction mixture with the anion exchanger, acetone, water and part of phenol are removed by distillation while the residue, containing from 25 to 35 % bisphenol A, is cooled prior to being sent to suspension crystallization and filtration or centrifugation in order to separate the bisphenol A-phenol adduct. The separated adduct is then washed with a stream of phenol and is recrystallized from a phenolic solution which, in part or entirety, prior to being used for recrystallization, is contacted in the presence of 1 - 5 % acetone with an acid ion-exchange resin in which 5 - 30 % acid groups are neutralized with 2,2-dimethylthiazolidine or cysteamine and with the anion exchanger whereas the recrystallized adduct, after being separated by filtration or centrifugation, is sent to a distillation system in order to recover bisphenol A and a distillate while the post-crystallization liquor is sent to the reaction Step I.

In yet another preferable embodiment of the present invention, the resulting post-reaction mixture is contacted with the anion exchanger and is then sent to a suspension crystallization unit, to obtain a crystalline bisphenol A-phenol adduct in the form of a suspension from which the adduct is obtained by filtration or centrifugation. The separated adduct is washed with a stream of phenol and recrystallized from the phenolic solution which, in part or entirety, prior to being used for recrystallization, is contacted in the presence of 1 - 5 % acetone with an acid ion-exchange resin in which 5 - 30 % acid groups are neutralized with 2,2-dimethylthiazolidine or cysteamine and with the anion exchanger whereas the recrystallized adduct, after being separated by way of filtration or centrifugation is sent to a distillation system in order to recover bisphenol A with high purity while the post-crystallization liquor is distilled to obtain a fraction composed mainly of acetone, water, and phenol, which fraction is distilled to separate therefrom acetone and phenol which are then sent back to the process, and water which is removed from the process. The dewatered post-crystallization liquor is sent either to the reaction Step I or Step III.

In another preferable embodiment of the present invention part of the dewatered post-crystallization liquor is distilled to separate a regenerated phenol fraction while the residue, containing bisphenol A and heavy byproducts, is thermally decomposed at a temperature in the range from 210 to 280 °C in the presence of basic catalysts and distilled, the resulting distillate is diluted with the phenol fraction and contacted with a macroporous cation exchanger at a temperature in the range from 65 to 95 °C and is then recycled to the process while the decomposition residue, that contains mainly heavy impurities, is removed from the process.

In another preferable embodiment of the invention, either molten polycarbonate or a mixture thereof with fractions that contain heavy byproducts arising in the process to obtain bisphenol A are thermally decomposed. The regenerated phenol is periodically subjected to fractional distillation to separate therefrom a fraction that contains isopropyl phenol, 4-tertbutylphenol and phenol, and the fraction is removed from the process.

### Best Mode for Carrying Out the Invention

**Example 1**

The first reactor in the series, of which the capacity is 85 m³ and which has filtration slot nozzles located at three heights and is packed with a solid bed of Purolite CT 124/3539 catalyst, is filled from the top with a feed mixture that contains 66.0 % phenol, 5.3 % acetone and 12.1 % bisphenol A, 16.3 % byproducts and 0.3 % water, and the mixture that flows downwards is contacted with the cation exchanger, located between the middle and top nozzles, at a temperature that is elevated from 61°C to 74°C. The reaction mixture from Step I which contains 19 % bisphenol A is made to flow through the nozzles to a 15-m³ tank equipped with a heat exchanger, where it is combined with part of the reaction mixture from the reaction Step II and, after being cooled to the temperature of 69°C, is made to flow through bottom nozzles to the reaction Step II which is effected in the catalyst bed between the bottom and middle nozzles.

In the reaction Step II, part of the reaction mixture at the temperature of 71°C is collected through the middle nozzles and is made to flow to the tank with the heat exchanger referred to above while the remaining part is removed outside the first reactor through nozzles that are located in the catalyst bed.

A portion of the reaction mixture resulting from the reaction Step II, at the temperature of 73° C, which is removed from the top of the first reactor, is made to flow to a second 15-m³ tank with a heat exchanger, acetone is added to obtain a concentration in the range 3 - 6 %, whereafter it is made to flow through the bottom nozzles to the second reactor to effect the reaction Step III in the catalyst bed between the bottom and middle nozzles. A portion of the reaction mixture from the reaction Step III is made to flow through the middle nozzles and removed to the second 15-m ₃ tank with a heat exchanger referred to above while the remaining portion of the reaction mixture is, after flowing through the solid catalyst bed is removed outside the reactor through the nozzles located in the middle part of the reactor. A portion of the reaction mixture is recycled to the second tank with the heat exchanger while the remaining portion of it is sent to a vacuum evaporator to distill, at a vacuum of 80 mm Hg at the temperature of 96 °C, the fraction that contains water, acetone and phenol while a dewatered residue containing 0.6 % water, is recycled through the top nozzles to the second reactor after adding an amount of acetone to obtain a concentration of 3 %, to effect the reaction Step IV while the reaction mixture is made to flow downwards through the cation exchanger located between the middle and top nozzles. The final resulting post-reaction mixture that contains 31 % bisphenol A and is removed through the nozzles located in the middle part of the second reactor is sent to a distillation column to obtain a light fraction with a water content of 0.3 % in the residue and then the residue is contacted with the anion exchanger Dowex 550A and sent to suspension crystallization and centrifugation. The resulting bisphenol A - phenol adduct is purified by recrystallization from the stream of phenol and then phenol is distilled off from the adduct in the evaporator and in a steam-stripper, to obtain bisphenol A with the purity of 99.94 % and coloration of 4 APHA.

The post-crystallization liquor and the recovered phenol and acetone are recycled to the process. A portion of the stream of the recovered phenol, prior to being sent to the recrystallization unit, is combined with acetone to obtain a concentration of 3.5 % and is contacted in two steps at a temperature in the range 57 - 72 °C, first with the resin Purolite CT 122 of which 21 % sulfonic groups were neutralized with 2,2-dimethylthiazolidine and then with the anion exchanger Dowex 550 A.

### Mode(s) for Carrying Out the Invention

**Example 2**

The reaction mixture obtained as described in Example 1 and containing 31 % bisphenol A is sent to the suspension crystallization and then to centrifugation in order to separate the bisphenol A-phenol adduct which is purified by recrystallization from the stream of phenol and by centrifugation.

The purified adduct is sent to an evaporator and a steam-stripper to obtain bisphenol A with the purity of 99.94 % and coloration of 3 APHA. The post-crystallization liquor is subjected to distillation to remove water to a concentration of 0.3 % while the remaining portion of it is recycled to the process along with the recovered phenol and acetone. A portion of the stream of the recovered phenol prior to being sent to recrystallization is combined with acetone to obtain a concentration of 3.5 % and is contacted in two steps at a temperature in the range 57 - 72 °C with the resin Purolite CT 122 of which 21 % sulfonic groups were neutralized with 2,2-dimethylthiazolidine, and then with the anion exchanger Dowex 550 A.

**Example 3**

A portion of the dewatered post-crystallization liquor obtained as described in Example 2 is subjected to distillation to obtain a regenerated phenol fraction which is 52 % of the feed stream. The remaining 48 % of the feed stream, containing mainly bisphenol A and heavy byproducts, is thermally decomposed by distillation at the temperature of 242 °C and vacuum of 4 mm Hg in the presence of a NaOH catalyst in the amount of 0.4 % by weight. The residue after the catalytic decomposition which contains mainly heavy impurities is removed from the process while the distillate is diluted with the phenolic fraction in the ratio of 3.5 : 1 and is then contacted in a continuous manner at the temperature of 75 °C with a solid bed of the macroporous cation exchanger Amberlyst 16 in the amount of 3 m³ in a 5-m³ tank reactor. The resulting mixture that contains 23 % bisphenol A is sent to a two-step suspension crystallization and centrifugation, while bisphenol A with the purity of 99.94 % and color 4 APHA is recovered from the resulting adduct as described in Example 2.

**Example 4**

A mixture, containing mainly heavy byproducts arising in the synthesis of bisphenol A and a molten polycarbonate, is thermally decomposed by distillation at the temperature of 268 °C and at a vacuum of 4 mm Hg in the presence of a NaOH catalyst in the amount of 0.6 % by weight. The residue after the catalytic decomposition is removed from the process while the distillate is diluted with the phenolic fraction in the ratio of 3 : 1 and is then contacted in a continuous manner at the temperature of 70 °C with a solid bed of the macroporous cation exchanger Amberlyst 16 in the amount of 3 m³ in a 5-m³ tank reactor. The resulting mixture that contains 26 % bisphenol A is sent to a suspension crystallization and centrifugation, while bisphenol A with the purity of 98.2 % and color 25 APHA is recovered from the resulting adduct by distillation.

## Claims

1. A method to obtain polycarbonate-grade bisphenol A from phenol and acetone in the presence of an acid ion-exchanger catalyst, in two or four reaction steps **characterized in that** two reaction steps are effected in a single reactor that is divided into reaction zones by means of sets of filtration-injection nozzles which are located at not less than three heights, in Step I the reaction being made to occur in the upper zone of the reactor while the reaction mixture is made to flow downwards, to obtain an increase in the concentration of bisphenol A by 2 - 18 %, while Step II is made to occur in the lower zone of the reactor while the reaction mixture is made to flow upwards [in the reactor] so that the difference in the concentrations between the inlet to the reaction zone and the outlet: is 0.1 - 0.6 % for water, is a maximum of 3 % for acetone, is 1 - 10 % for bisphenol A while the difference in temperatures between the inlet to the reaction zone and the outlet is a maximum of 12 °C, preferably a maximum of 3 °C.

2. A method as claimed in Claim 1 **characterized in that** the four reaction steps are effected in two reactors, Steps I and II being effected in the first reactor as claimed in Claim 1 while Step III proceeds in the lower zone of the second reactor that is divided into reaction zones by means of sets of filtration-injection nozzles which are located at not less than three heights while the reaction mixture is made to flow upwards in the reactor so that the difference in the concentrations between the inlet to the reaction zone and the outlet: is 0.1 - 0.6 % for water, is a maximum of 2 % for acetone, is 1 - 8 % for bisphenol A while the difference in temperatures between the inlet to the reaction zone and the outlet is a maximum of 10 °C, preferably a maximum of 2 °C, whereupon part of water is removed from the reaction mixture after Step III by distillation to obtain a concentration of not more than 1.3 %, preferably not more than 0.8 %, while adding acetone thereto to obtain a concentration in the range from 2 to 4 % and is contacted, in Step IV, at a temperature in the range 70 - 90 °C with the cation exchanger which is located in the upper zone of the second reactor while the reaction mixture is made to flow downwards.

3. A method as claimed in Claim 1 **characterized in that** the four reaction steps are effected in two reactors, Steps I and II being made to occur in the first reactor as claimed in Claim 1, Step III proceeds in the upper zone of the second reactor that is divided into reaction zones by means of sets of filtration-injection nozzles which are located at not less than three heights while the reaction mixture is made to flow downwards, to obtain an increase in the concentration of bisphenol A by 1 - 10 % whereas Step IV proceeds in the lower zone of the second reactor while the reaction mixture is made to flow upwards in the reactor so that the difference in the concentrations between the inlet to the reaction zone and the outlet: is 0.1 - 0.6 % for water, is a maximum of 2 % for acetone, is 1 - 8 % for bisphenol A, while the difference in temperatures between the inlet to the reaction zone and the outlet is a maximum of 10 °C, preferably a maximum of 2 °C.

4. A method as claimed in Claim 1 **characterized in that** the four reaction steps are effected in two reactors, Steps I and II being made to occur in the first reactor as claimed in Claim 1, Step III proceeds in the lower zone of the second reactor that is divided into reaction zones by means of sets of filtration-injection nozzles which are located at not less than three heights while the reaction mixture is made to flow upwards [in the reactor] so that the difference in the concentrations between the inlet to the reaction zone and the outlet: is 0.1 - 0.6 % for water, is a maximum of 2 % for acetone, is 1 - 8 % for bisphenol A, while the difference in temperatures between the inlet to the reaction zone and the outlet is a maximum of 10 °C, preferably a maximum of 2 °C, whereas Step IV proceeds in the upper zone of the second reactor while the reaction mixture is made to flow downwards at a temperature in the range 74 - 90 °C.

5. A method as claimed in Claims 1 - 4 **characterized in that** the reactor is fed from the top with a dewatered the post-crystallization liquor resulting from centrifugation or filtration of the bisphenol A-phenol adduct.

6. A method as claimed in Claims 1 - 4 **characterized in that** the reactor is fed from the top with a post-reaction mixture from another reactor.

7. A method as claimed in Claims 1 - 4 **characterized in that** the reactor is fed from the top with a portion of the feed mixture to the reaction Step II.

8. A method as claimed in Claims 1 - 4 **characterized in that** the reactor is fed from the top with a portion of the feed mixture to the reaction Step III.

9. A method as claimed in Claims 1 - 4 **characterized in that** the resulting post-reaction mixture, containing from 23 to 30 % bisphenol A, is sent to a distillation system to distill off acetone, water, and phenol whereby a residue in the form of crude bisphenol A, containing not more than 5 %, preferably not more than 1 % phenol, is obtained and is sent to a falling-film melt fractional crystallization unit, to obtain bisphenol A with high purity and a residue that contains bisphenol A, its isomers and other byproducts, whereas the distillate that contains acetone, water and phenol is separated by distillation to obtain acetone and phenol which are sent back to the process, and water which is removed from the process.

10. A method as claimed in Claims 1 - 4 **characterized in that** the resulting post-reaction mixture is contacted with the anion exchanger, acetone, water and part of phenol are removed therefrom by distillation while the residue, containing from 25 to 35 % bisphenol A, is cooled prior to being sent to the suspension crystallization unit and to filtration or centrifugation in order to separate the bisphenol A-phenol adduct which is then washed with a stream of phenol and recrystallized from the phenolic solution which, in part or entirety, prior to being used for recrystallization, is contacted in the presence of 1 - 5 % acetone with an acid ion-exchange resin in which 5 - 30 % acid groups are neutralized with 2,2-dimethylthiazolidine or cysteamine and with the anion exchanger while the recrystallized adduct, separated by way of filtration or centrifugation, is sent to the distillation system in order to recover bisphenol A and a distillate therefrom, while the post-crystallization liquor is sent to the reaction Step I or Step III.

11. A method as claimed in Claims 1 - 4 **characterized in that** the resulting post-reaction mixture is contacted with the anion exchanger and is then sent to a suspension crystallization unit to obtain a crystalline bisphenol A-phenol adduct in the form of a suspension which is subjected to filtration or centrifugation to separate the adduct, which is washed with a stream of phenol and recrystallized from the phenolic solution which, in part or entirety, prior to being used for recrystallization, is contacted in the presence of 1 - 5 % acetone with an acid ion-exchange resin in which 5 - 30 % acid groups are neutralized with 2,2-dimethylthiazolidine or cysteamine and with the anion exchanger while the recrystallized adduct, separated by way of filtration or centrifugation, is sent to the distillation system in order to recover bisphenol A with high purity while the post-crystallization liquor is distilled to obtain a fraction composed mainly of acetone, water, and phenol, and which fraction is separated by distillation into acetone and phenol to be recycled to the process, and water which is removed from the process while the dewatered post-crystallization liquor is sent either to the reaction Step I or Step III.

12. A method as claimed in Claims 10 and 11 **characterized in that** a portion of the dewatered post-crystallization liquor is thermally decomposed at a temperature in the range from 210 to 280 °C in the presence of basic or acid catalysts and vacuum-distilled, and the resulting distillate is diluted with a phenolic fraction and contacted with a macroporous cation exchanger at a temperature in the range from 65 to 95 °C, whereafter bisphenol A is recovered by way of suspension crystallization and distillation or by distillation while the thermal decomposition residue, containing mainly heavy products and polymers, is removed from the process.

13. A method as claimed in Claim 12 **characterized in that** either molten polycarbonate or a mixture thereof with fractions that contain heavy byproducts from the process to obtain bisphenol A is thermally decomposed.

14. A method as claimed in Claim 12 **characterized in that** the regenerated phenol is periodically subjected to fractional distillation to separate therefrom the fraction that contains isopropylphenol, 4-tertbutylphenol and phenol, and which is removed from the process. 15.
